# EUROPEAN PATENT APPLICATION

(11) **EP 1 452 188 A1**
(43) Date of publication of application: **01.09.2004**
(21) Application number: 02803922.0
(22) Date of filing: 25.11.2002
(51) Int. Cl.: A61L 9/01, A61K 7/075

(54) **DEODORANT AND COSMETIC SHAMPOO PREPARATION CONTAINING THE SAME**

(30) Priority: 26.11.2001 JP 2001359183
(71) Applicant: Yugen Kaisha Okada Giken, Kakogawa-shi, Hyogo 675-0062 (JP)
(72) Inventor: OKADA, Toru, Kakogawa-shi, Hyogo 675-0062 (JP)
(74) Representative: Keck, Stephan
(86) International application number: PCT/JP2002/012252
(87) International publication number: WO 2003/045449

(57) **Abstract**

A deodorant which is suitable for removal of various odors in daily life, especially for removing a chemical odor remaining in the hair after a permanent wave treatment. It less irritates the hair and skin and is suitable for incorporation into a shampoo, treatment agent, etc. The deodorant contains a deodorizing ingredient selected among the following (a) to (d):
(a) a hydroxy acid salt of a divalent metal
(b) a fatty acid salt of a divalent metal
(c) an oxide of a divalent metal and a hydroxy acid, fatty acid, or chelating agent
(d) an inorganic acid salt of a divalent metal and a hydroxy acid, fatty acid, or chelating agent.

## Description

### TECHNICAL FIELD

This invention relates.to a deodorant. More particularly the present invention is a deodorant that works against various basic odors (such as ammonia) and acidic odors (such as mercaptans), and is used to eliminate daily odors such as body odor, excrement odor, and other indoor odors. The deodorant of the present invention is particularly effective at removing the unpleasant odors of chemical agents that remain behind after head hair has undergone a permanent wave treatment. The present invention also relates to a head hair washing product, and particularly a shampoo or treatment agent (rinse), containing the above-mentioned deodorant. The deodorant of the present invention also has an antibacterial action.

### BACKGROUND ART

As the standard of living here [in Japan] has risen, there has been growing interest in environmental sanitation and in preventing and eliminating odors. Methods known in the past for reducing malodorous ingredients included masking unpleasant odors with a powerful perfume; physical methods such as adsorbing odors with activated charcoal, alumina, zeolite, or another such porous adsorbent; chemical methods such as catalytic combustion, oxidation of the odor ingredients with ozone, and neutralization with chemicals; and biochemical methods such as decomposing odor ingredients with bacteria.

For example, when head hair is subjected to a permanent wave treatment at a beauty salon or the like, ammonium thioglycolate and other such chemicals are used to make the wave last longer. Almost all of the chemical is removed upon completion of the permanent treatment by rinsing with water or performing an acid treatment (pH of about 3) or reduction treatment. Nevertheless, a tiny amount of ammonium thioglycolate ingredient remains on the head hair, and this leaves the head hair with a distinctive odor for 2 to 3 days.

Unpleasant odors that actually pose a problem in daily life, such as the residual odor after a permanent treatment, include basic odors, acidic odors, and odors that are a mixture of these, and are made up of a variety of ingredients. There is a need for a deodorant that is effective against all of these unpleasant odor ingredients.

It is an object of the present invention to provide a deodorant that is suited to the removable of various odors encountered in daily life, and especially the chemical smell that remains on head hair after a permanent wave treatment. The inventor conducted various investigations aimed at solving this problem.. As a result, the inventor learned that using a deodorizing antibacterial agent comprising a combination of a divalent metal ion such as a copper ion and a hydroxy acid, a fatty acid or another such organic acid ingredient, or a specific chelating agent allows odors after a permanent treatment to be effectively eliminated, and causes minimal irritation to the head hair and skin.

### DISCLOSURE OF THE INVENTION

The first invention of the present application is a deodorant containing a deodorizing ingredient selected from among the following (a) to (d):
(a) a hydroxy acid salt of a divalent metal
(b) a fatty acid salt of a divalent metal
(c) an oxide of a divalent metal and a hydroxy acid, fatty acid, or chelating agent
(d) an inorganic acid salt of a divalent metal and a hydroxy acid, fatty acid, or chelating agent.

These divalent metals are preferably copper, zinc and/or iron. The second invention of the present application is a head hair washing product such as a shampoo or treatment agent containing the deodorant. The third invention of the present application is a head hair treatment method, wherein the head hair is first given a permanent wave, and then washed using the head hair washing product. The fourth invention of the present application is a deodorizing antibacterial substrate, produced by applying the deodorant to the surface and/or the interior of a fibrous material, granular material, paper, nonwoven cloth, woven cloth, porous substance, or polymer film. The deodorant may further contain a betaine compound, a carbonyl compound, and/or α-olefin/maleic anhydride copolymer.

### BEST MODE FOR CARRYING OUT THE INVENTION

### Detailed Description of the Invention

The active ingredient in the deodorant of the present invention against acidic odors such as amines and mercaptans is believed to be a divalent metal ion. The active ingredient against basic odors such as ammonia in this deodorant is believed to be a hydroxy acid radical, fatty acid radical, or the like.

### (a) Metal compound

The divalent metal ion can be introduced in any of various forms, but in particular it can be introduced as a divalent metal oxide, inorganic metal salt (such as a sulfate), or organic acid salt (such as a hydroxy acid metal salt or fatty acid metal salt). These divalent metal ions are mainly capable of eliminating amine odors, mercaptan odors, and other such acidic odors.

The amount in which the metal compound is contained in the deodorant may be suitably selected according to the form of the deodorant, but is usually 0.01 to 30 wt%, and preferably 0.1 to 5 wt%, with respect to the total amount of deodorant. In a head hair washing product, [this content] is 0.01 to 10 wt%, and preferably 0.05 to 5 wt%.

### Metal oxide

Examples of metal oxides that' can be used in the deodorizing antibacterial agent of the present invention include metal oxides that provide divalent metal ions in a solution in the presence of a hydroxy acid or fatty acid such as copper oxide, zinc oxide, and iron oxide.

### Inorganic metal salt

Examples of inorganic salts of divalent metals that can be used in the deodorizing antibacterial agent of the present invention include sulfates and nitrates. Specific examples include copper sulfate, copper nitrate, zinc sulfate, and iron sulfate. These provide divalent metal ions in a solution in the presence of a hydroxy acid or fatty acid.

### Hydroxy acid metal salt

Of the various organic acid salts, examples of hydroxy acid metal salts include hydroxy acid copper salts, hydroxy acid zinc salts, and hydroxy acid iron salts. Examples of the hydroxy acid ingredient of these hydroxy acid metal salts include gluconic acid and other such aldonic acids, as well as saccharic acid and other such saccharide oxides, and various other hydroxy acids such as ascorbic acid, dehydroascorbic acid, lactic acid, malic acid, tartaric acid, citric acid, glycolic acid, hydroxybenzoic acid, gallic acid, mandelic acid, and tropic acid. Of these, gluconic acid, malic acid, and citric acid are preferable, and gluconic acid is particularly favorable. Therefore, copper gluconate and zinc gluconate are preferred.

A commercially available hydroxy acid metal salt can be used, but a combination of a hydroxy acid such as gluconic acid, saccharic acid, or ascorbic acid and one of the above-mentioned metal oxides (such as copper oxide, zinc oxide, and iron oxide) or an inorganic metal salt may be used instead. When a hydroxy acid ingredient is thus present, it stabilizes the divalent metal ion, yielding a higher metal ion concentration. The hydroxy acid metal salts may be used singly or in combinations of two or more types.

Gluconic acid metal salts such as copper gluconate and zinc gluconate have good deodorizing action against unpleasant odors from both ammonia and mercaptans, and give an excellent deodorant and antibacterial agent.

### Fatty acid metal salt

Of the various organic acid salts, the fatty acid metal salts can be metal salts of saturated or unsaturated fatty acids with 1 to 30 carbons. Specific favorable examples include metal salts of saturated fatty acids such as acetic acid, propionic acid, undecylenic acid, palmitic acid, lauric acid, myristic acid, palmitic acid [*sic*], stearic acid, and behenic acid; and of unsaturated fatty acids such as oleic acid, sorbic acid, linoleic acid, linolenic acid, ricinoleic acid, and arachidonic acid. Examples of these metal salts include zinc, copper, iron, molybdenum, cobalt, aluminum, titanium, manganese, nickel, and silver, with copper, zinc, and iron salts being particularly preferred.

A commercially available fatty acid metal salt can be used, but a combination of a fatty acid such as palmitic acid or stearic acid and one of the above-mentioned metal oxides (such as copper oxide, zinc oxide, and iron oxide) or an inorganic metal salt may be used instead. This fatty acid metal salt exhibits an excellent deodorizing action against basic and acidic odors from ammonia, mercaptans, and so forth. These fatty acid metal salts may be used singly or in combinations of two or more types.

### Chelating agent

The chelating agent is preferably an amino acid type of chelating agent, and specific examples include hydroxyethylethylenediamine triacetate, ethylenediamine tetraacetate, nitrilotriacetate, diethylenetriamine pentaacetate, triethylenetetraamine hexaacetate, tetrasodium dicarboxymethylglutamate, and dihydroxyethylglycine and the like. With the present invention, adding a chelating agent allows the metal component concentration in the deodorant to be kept high in the solution. The amount in which this agent is contained is 0.1 to 50 weight parts, and preferably 1 to 20 [weight parts], weight parts per 100 weight parts of deodorant.

### (b) Betaine acid

With the deodorant of the present invention, it is preferable to use a betaine compound along with the above-mentioned deodorizing ingredient in order to enhance the contact efficiency with the odor ingredient and to improve solubility in a solution of the deodorizing ingredient. Examples of such betaines include cocoamidopropyl betaine, 2-alkyl-N-carboxymethyl-N-hydroxyethylimidazolinium betaine, 2-undecyl-N-carboxymethyl-N-hydroxyethylimidazolinium betaine, lauryldimethylaminoacetic acid betaine, N-lauroyl-N-carboxymethyl-N-hydroxyethylimidazolinium, lauric acid amidopropyl betaine, and various other betaines. The amount in which this betaine is contained is 10 to 300 weight parts, and preferably 40 to 200 weight parts, per 100 weight parts of deodorizing ingredient.

### (c) Carbonyl compound

A ketocarboxylic acid, aldehydic acid, or other such carbonyl compound may be further contained with the present invention. Examples of this carbonyl compound include glyceraldehyde, pyruvic acid, levulinic acid, aldehyde nonanoic acid, glyoxylic acid, and aldehydic acid..Adding this compound improves the removal of basic and acidic odors.

The amount in which this carbonyl compound is contained is 5 to 100 weight parts, and preferably 10 to 200 weight parts, per 100 weight parts of gluconic acid metal salt or other deodorizing ingredient.

### (d) α-olefin/maleic anhydride copolymer

The deodorant of the present invention may further contain an α-olefin/maleic anhydride copolymer in order to improve the removal of basic and acidic odors. Examples of such.resins include methyl vinyl ether/maleic anhydride copolymers and isobutylene/maleic anhydride copolymers. Typical examples of methyl vinyl ether/maleic anhydride copolymers include products of various molecular weight available from Daicel Chemical Industries under the product name of VEMA (an alternate copolymer of methyl vinyl ether and maleic anhydride). Typical examples of the above-mentioned isobutylene/maleic anhydride copolymers include products of various molecular weight available from Kuraray Co., Ltd. under the product name of Isobam (an alternate copolymer of isobutylene and maleic anhydride).

A reaction product obtained by reacting one of these α-olefin/maleic anhydride copolymers with a copper compound (such as copper oxide) and/or a zinc compound (such as zinc oxide) or the like may also be added. Copper, zinc, or another such metal is bonded to at least some of the maleic anhydride structural units in this copolymer. The amount in which this copolymer is contained is 1 to 300 weight parts, and preferably 5 to 100 weight parts, per 100 weight parts of gluconic acid metal salt or other deodorizing ingredient.

The deodorant of the present invention exhibits excellent adsorption power and deodorizing effect against residual odors after a permanent treatment, as well as ammonia or other such basic odors and mercaptans or other such acidic odors that pose a problem in daily life. Furthermore, if a betaine, fatty acid metal salt, inorganic metal compound, carbonyl compound, or the like is added, substances that cause unpleasant odors, such as hydrogen sulfide and mercaptans, will be absorbed even more effectively.

### (Manufacturing method)

In the manufacture of the deodorant of the present invention, a betaine, carbonyl compound, maleic anhydride copolymer, or the like can be used as needed in liquid form, either dispersed or dissolved in a suitable solvent, with the divalent metal compound such as a metal oxide, inorganic metal salt, or organic acid metal salt that serves as the deodorizing ingredient.

There are no particular restrictions on the solvent used in the preparation of the deodorant, as long as it will dissolve the various ingredients, but examples include water; alcohols such as methyl alcohol and ethyl alcohol; and dimethylformamide and dimethyl sulfoxide. The amount in which the solvent is. used is preferably 2 to 50 weight parts per 100 weight parts of deodorizing ingredient.

The deodorant of the present invention can be added to head hair washing products and various other head hair treatment products, such as shampoo, treatment agents, hair spray, hair milk, and blow-drying agents, or can be added to other cosmetic products. To manufacture a head hair treatment product or a head hair washing product such as a shampoo or treatment [agent], the above-mentioned deodorant should be added to and mixed with known head hair washing product ingredients or head hair treatment ingredients. In addition to the deodorant, these head hair washing agents and head hair treatment agents may contain known surfactants, perfumes, colorants, oils, antibacterial agents, and so forth according to the formulation of the product.

This deodorant may also be used to coat the surface of fibers or a film, or mixed into a film, for example, to deodorize the surrounding environment. Specifically, the deodorant of the present invention may be applied as a layer over a substrate in any of various forms, such as a sheet or yarn, or may be used to impregnate [such a substrate], which increases the surface area of the deodorant composition and improves its deodorizing action. This substrate can be nonwoven cloth, woven cloth, a fibrous material such as yarn or monofilaments, a granular material, paper, or a porous substance such as zeolite, sepiolite, diatomaceous earth, or activated charcoal: These materials can be used singly or in combinations.

When the deodorant is applied to a substrate, various kinds of adhesive colorant may also be added in order to adjust the hand or appearance of the product, or to apply an adhesive coating to a substrate composed of a sheet- or yarn-form composition. A suitable polymer compound can be used as this substrate, examples of which include vinyl acetate/acrylic copolymers, polyvinyl alcohol, urethane resin, ethylene/vinyl acetate copolymers, and carboxymethyl cellulose. These may be used singly or in mixtures.

In other usage modes of the deodorant of the present invention, it can be dispersed and mixed into various polymer compounds, or it can be alternately dissolved into a base polymer to create a polymer blend, and this mixture may be molded into a sheet, film, or the like.

### Examples

The present invention will now be described in specific terms through examples. All added amounts in the examples and comparative examples are in weight percent.

### [Example 1]

1.0 g of copper oxide and 5.0 g of ethylenediaminetetraacetate (EDTA) were dispersed and stirred in enough water for the total to be 100 g. The deodorizing liquid thus obtained was sprayed onto tissue paper (10 × 10 cm) to impregnate the paper (adhering amount: 1 mL). This paper was dried for 1 hour at 80°C to obtain a deodorizing paper.

This deodorizing paper was placed in a Teflon® container (5 L volume), and nitrogen gas containing 5 L of ammonia (500 ppm) and 5 L of hydrogen sulfide (100 ppm) was charged into the container. The change in gas concentration in the container over time was measured, the results of which are given in Table 1.

### [Examples 2 to 4]

Other than preparing deodorants using the ingredients shown in Table 1, the various deodorants were manufactured in the same manner as in Example 1. Each was sprayed onto tissue paper to obtain deodorizing paper. A deodorizing test was conducted in the same manner as in Example 1, the results of which are given in Table 1.

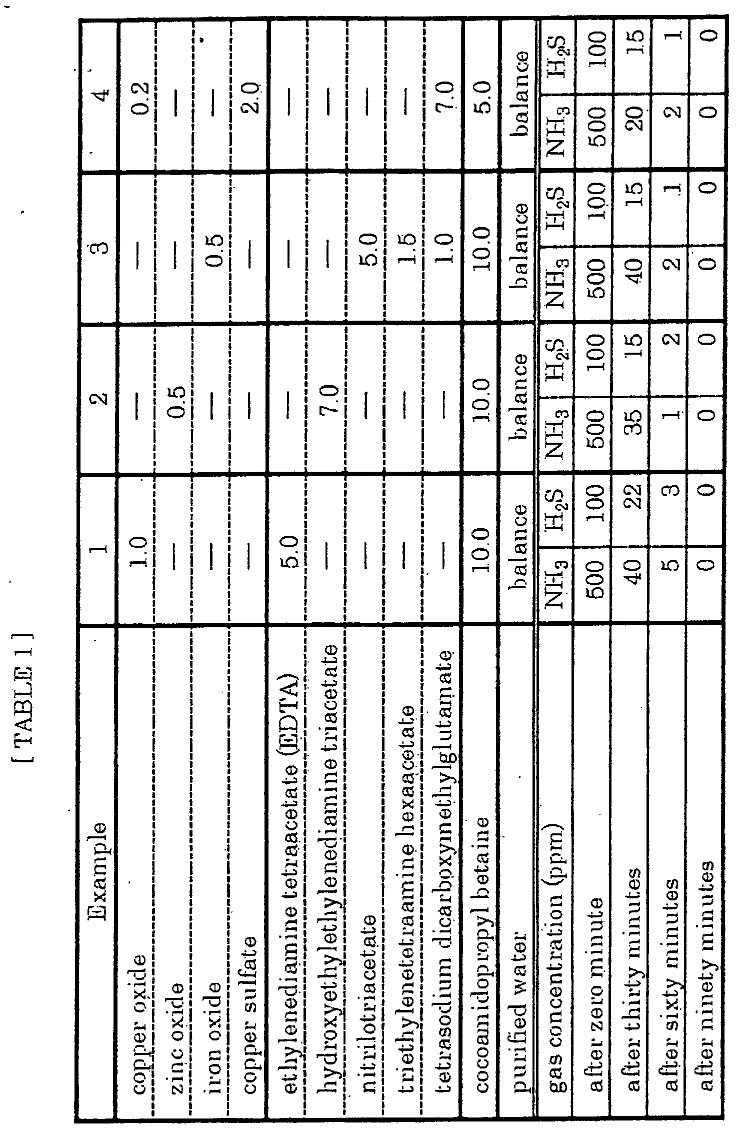

### [Example 5]

4.0 g of copper gluconate was dispersed and stirred in enough water for the total to be 100 g. The deodorizing liquid thus obtained was sprayed onto tissue paper (10 × 10 cm) to impregnate the paper (adhering amount: 1 mL). This paper was dried for 1 hour at 80°C to obtain a deodorizing paper. This deodorizing liquid^{[6]} was placed in a Teflon® container (5 L volume), and nitrogen gas prepared to^{[7]} 5 L of ammonia (500 ppm) and 5 L of hydrogen sulfide (100 ppm), or these and 5 L of acetic acid (20 ppm), was charged into the container. The change in gas concentration in the container over time was measured, the results of which are given in Table 2.

### [Examples 6 to 14]

Other than preparing deodorants using the ingredients shown in Tables 2 and 3, the various deodorants were manufactured in the same manner as in Example 5. Each was sprayed onto tissue paper to obtain deodorizing paper. The products were evaluated in the same manner as in Example 5, the results of which are given in Tables 2 and 3.

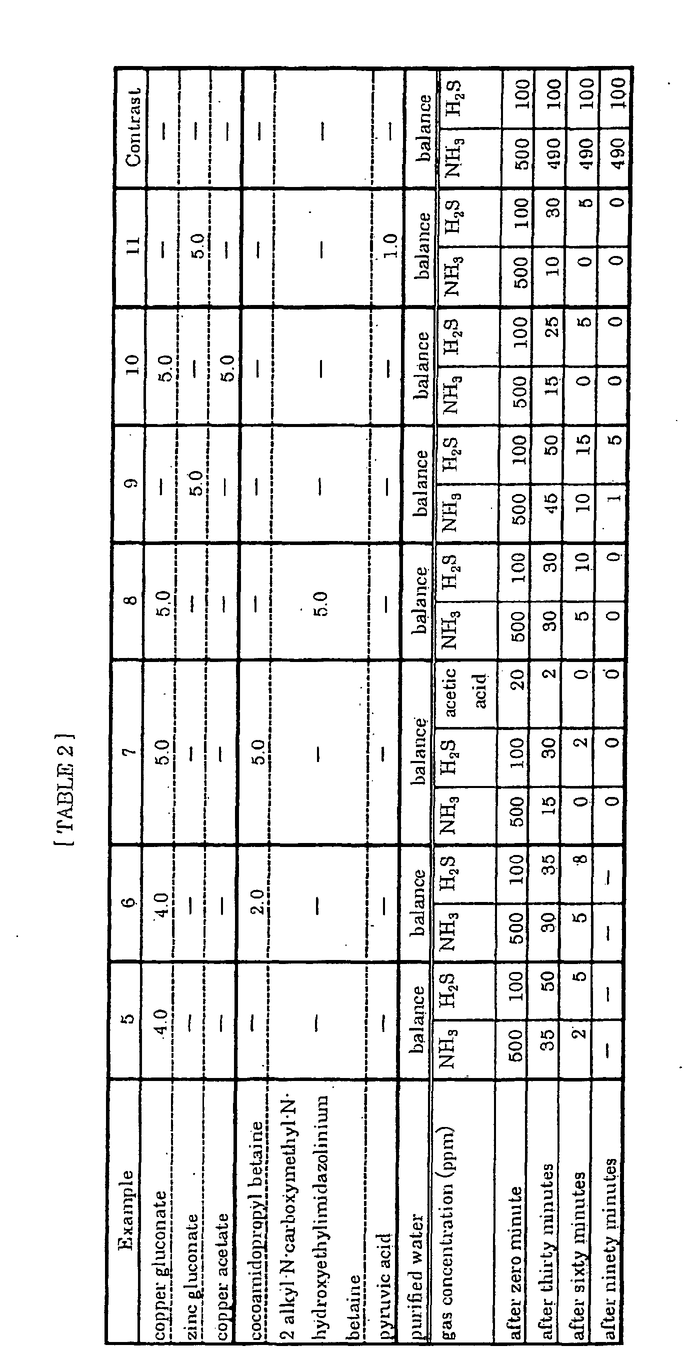

**[TABLE 3]**

| Example | 12 | | 13 | | 14 | |
|---|---|---|---|---|---|---|
| copper oxide | 0.5 | | ― | | 0.5 | |
| zinc oxide | ― | | 0.2 | | ― | |
| saccharic acid | 5.0 | | ― | | ― | |
| gallic acid | ― | | ― | | 4.0 | |
| ascorbic acid | ― | | 5.0 | | ― | |
| cocoamidopropyl betaine | ― | | 10.0 | | 10.0 | |
| purified water | balance | | balance | | balance | |

| gas concentration (ppm) | NH₃ | H₂S | NH₃ | H₂S | NH₃ | H₂S |
|---|---|---|---|---|---|---|
| after zero minute | 500 | 100 | 500 | 100 | 500 | 100 |
| after thirty minutes | 30 | 25 | 35 | 20 | 25. | 40 |
| after sixty minutes | 1 | 2 | 5 | 2 | 5 | 10 |
| after ninety minutes | 0 | 0 | 0 | 0 | 0 | 0 |

### [Example 15: shampoo]

| Deodorant composition | wt% |
|---|---|
| copper gluconate | 5 |
| 2-alkyl-N-carboxymethyl-N-hydroxyethyl-imidazolinium betaine | 10 |
| pyruvic acid | 1 |
| purified water | balance' |

| Shampoo ingredients | wt% |
|---|---|
| triethanolamine alkylsulfate | 25 |
| polyoxyethylene alkyl (12, 13) ether sodium sulfate | 15 |
| coconut oil fatty acid diethanolamide | 5 |
| table salt | 1 |
| propylene glycol | 5 |
| purified water | balance |

The above ingredients were mixed and stirred to prepare a deodorant and a shampoo base. Next, 2.5 g of the deodorant was added to 97.5 g of this shampoo base to prepare a deodorant-containing shampoo. This shampoo was used for the final head hair washing after an ordinary permanent treatment (including an ammonium thioglycolate treatment). After shampooing, an organoleptic test was conducted to determine whether any ammonium thioglycolate odor remained. This test was conducted with 20 people who used the shampoo of Example 15 and with 20 who did not. None of the people who used the shampoo of Example 15 noticed any chemical odor, whereas all of the people who did not use this shampoo noticed a residual chemical odor.

### [Examples 16 to 29: shampoo]

Other than changing the deodorant in Example 15 to the deodorants of Examples 1 to 14, the shampoos of Examples 16 to 29 were manufactured in the same manner [as in Example 15]. The products were evaluated in the same manner as in Example 15, whereupon no residual odor was detected, and good results were obtained in every case.

### [Example 30: treatment agent]

| Head hair treatment ingredients | wt% |
|---|---|
| quaternary copolymer of vinylpyrrolidone/ dimethylaminoethyl methacrylate | 25 |
| ammonium cetyltrimethyl bromide | 10 |
| purified water | balance |

The above ingredients were mixed and stirred to prepare a deodorant and a treatment base. 2.5 g of the deodorant prepared in Example 15 was added to 97.5 g of this treatment agent base to prepare a deodorant-containing treatment agent. This treatment agent was used in treatment following an ordinary permanent treatment (including an ammonium thioglycolate treatment) and washing of the head hair. The same organoleptic test as in Example 15 was conducted to see if there was any residual ammonium thioglycolate odor after the head hair treatment, whereupon no residual odor was detected, and the same good results were obtained.

### INDUSTRIAL APPLICABILITY

The deodorant of the present invention causes minimal irritation or other adverse effects to the skin or head hair of humans, and has a particularly outstanding effect in the removal of an ammonia odor from head hair that has undergone a permanent treatment.

## Claims

1. A deodorant containing a deodorizing ingredient selected from among the following (a) to (d):
(a) a hydroxy acid salt of a divalent metal
(b) a fatty acid salt of a divalent metal
(c) an oxide of a divalent metal and a hydroxy acid, fatty acid, or chelating agent
(d) an inorganic acid salt of a divalent metal and a hydroxy acid, fatty acid, or chelating agent.

2. The deodorant according to Claim 1, wherein the divalent metal is at least one type of metal selected from among copper, zinc, and iron.

3. The deodorant according to Claim 1, wherein the deodorizing ingredients (a) to (d) are as follows:
(a) a hydroxy acid copper salt, hydroxy acid zinc salt, or hydroxy acid iron salt
(b) copper acetate and copper propionate
(c) copper oxide, zinc oxide, or iron oxide and a hydroxy acid, fatty acid, or chelating agent
(d) copper sulfate and a hydroxy acid, fatty acid, or chelating agent.

4. The deodorant according to Claim 1, further containing a betaine compound and/or a carbonyl compound.

5. A head hair washing product containing the deodorant according to any of Claims 1 to 4.

6. A head hair treatment method, wherein the head hair is first given a permanent wave, and then washed using the head hair washing product according to Claim 5.

7. A deodorizing antibacterial substrate, produced by applying the deodorant according to any of Claims 1 to 4 to the surface and/or the interior of a fibrous material, granular material, paper, nonwoven cloth, woven cloth, porous substance, or polymer film.
